# EUROPEAN PATENT SPECIFICATION

(11) **EP 1 404 367 B1**
(45) Date of publication and mention of the grant of the patent: **25.08.2010**
(21) Application number: 02745565.8
(22) Date of filing: 02.07.2002
(51) Int. Cl.: A61K 39/395

(54) **USE OF POLYCLONAL ANTI-HIV GOAT SERUM AS A THERAPEUTIC AGENT**
VERWENDUNG VON POLYKLONALEN ANTI-HIV ZIEGENSEREN ALS THERAPEUTISCHES MITTEL
UTILISATION D'UN SERUM POLYCLONAL DE CHEVRE ANTI-VIH COMME AGENT THERAPEUTIQUE

(30) Priority: 02.07.2001 GB 0116151; 29.11.2001 GB 0128638; 28.01.2002 GB 0201896; 28.03.2002 GB 0207509
(43) Date of publication of application: 07.04.2004
(73) Proprietor: Aimsco Limited, East Sussex BN21 4RL (GB)
(72) Inventor: DALGLEISH, Angus G., Cheam, Surrey SU12 7PP (GB); CADOGAN, Martin, Hendon London NW4 1LN (GB); HEENEY, Jonathan, NL-2275 BX Voorburg (NL); WHITE, Stanley D., T., Hardwick, MA 01037 (US)
(74) Representative: Ruffles, Graham Keith
(86) International application number: PCT/GB2002/003037
(87) International publication number: WO 2003/004049

(56) References cited:
- EP-A- 1 156 060
- EP-A- 1 156 062
- WO-A-00/12560
- WO-A-00/71160
- WO-A-02/07760
- WO-A-96/20215
- WO-A-96/40941
- WO-A-97/02839
- WO-A-98/13066
- SAIFUDDIN M ET AL: "Expression of MHC class II in T cells is associated with increased HIV-1 expression." CLINICAL AND EXPERIMENTAL IMMUNOLOGY, vol. 121, no. 2, August 2000 (2000-08), pages 324-331, XP002227061 ISSN: 0009-9104
- DATABASE WPI Section Ch, Week 199044 Derwent Publications Ltd., London, GB; Class B04, AN 1990-331416 XP002227065 & JP 02 237935 A (BIO KAGAKU KENKYUSH), 20 September 1990 (1990-09-20)
- JONES-BRANDO LORRAINE V ET AL: "Metabolites of the antipsychotic agent clozapine inhibit the replication of human immunodeficiency virus type 1." SCHIZOPHRENIA RESEARCH, vol. 25, no. 1, 1997, pages 63-70, XP002227062 ISSN: 0920-9964
- KUTSCH O ET AL: "Induction of the chemokines interleukin-8 and IP-10 by human immunodeficiency virus type 1 Tat in astrocytes." JOURNAL OF VIROLOGY, vol. 74, no. 19, October 2000 (2000-10), pages 9214-9221, XP002227063 ISSN: 0022-538X
- SILVESTRIS FRANCO ET AL: "Autoreactivity in HIV-1 infection: The role of molecular mimicry." CLINICAL IMMUNOLOGY AND IMMUNOPATHOLOGY, vol. 75, no. 3, 1995, pages 197-205, XP002227064 ISSN: 0090-1229
- SUTTON ET AL: "Hyperimmune goat serum (Aimspro): Hope or hype" JOURNAL OF CLINICAL NEUROSCIENCE, vol. 13, 2006, pages 1037-1038,
- DAY T.J.: "Comment on: "Follow-up study of hyperimmune goat serum (AIMSPRO) for amyotrophic lateral sclerosis" JOURNAL OF CLINICAL NEUROSCIENCE, vol. 16, 2009, pages 1509-1511,

## Description

The present invention relates to a therapeutic agent, in particular, but not exclusively, a therapeutic agent for the treatment of diseases involving a proliferative immune response.

### BACKGROUND OF THE INVENTION

WO 97/02839 relates to Viral Suppression, Treatment and Prevention of Viral Infections. It provides a method for producing neutralizing antibodies for the treatment of a viral infection in a patient, comprising the steps of:
a. exposing a mammal to a virus such that said mammal produces neutralizing antibodies to said virus and
b. collecting said neutralizing antibodies from said mammal. In the examples, an HIV vaccine designated AAV2 is obtained by mixing HIV virus with HIV neutralizing antibodies obtained from a goat.

WO 01/60156 relates to Neutralizing Antibody and Immunomodulatory Enhancing Compositions. It provides an immunomodulatory composition comprising:
heterologous antibodies specific for an antigen; and
an antigen, wherein the heterologous antibodies form a complex with the antigen for combination with a pharmaceutical carrier.
The examples are similar to those of WO 97/02839, and again an HIV vaccine designated AAV2 is obtained by mixing HIV virus with HIV neutralizing antibodies obtained from a goat.

WO 96/20215 describes the use of anti-MHC class II antibodies to interfere in the interaction between lipopolysaccharide (LPS) and its transducer molecule. This is said to be of benefit in the treatment of various inflammatory diseases and disorders. The publication describes experimental results obtained from in vitro systems, and from *in vivo* treatment of mice injected with LPS and pretreated with mouse anti-HLA class II antibodies.

WO 98/13066 describes the use of anti-MHC class II antibodies to inhibit T-cell independent B-cell function to prevent or reduce xenograft rejection.

WO 02/07760 is concerned with a Therapeutic Agent. It provides a method of preventing HIV infection or treating an individual infected with HIV, comprising the steps of
(1) exposure of goat immune system to HIV;
(2) purification of antibody from the goat after HIV challenge; and
(3) treatment of an individual with the antibody obtained in step 2 above.

In preliminary clinical trials based on the antibody product of WO 02/07760, patients with HIV have been treated successfully using serum from a goat after challenge with HIV.

Preferably the treatment employs a serum composition which can be obtained by a process involving raising effective antibodies in a goat, draining blood from the goat, demonstrating HIV neutralising capability in the drawn blood, removing solids from the blood, precipitating solids using supersaturated ammonium sulphate or other suitable precipitation agent, separating the precipitate, dissolving the precipitate in a suitable aqueous medium, and dialysing the solution with a cut-off of 5 to 50,000 Daltons, preferably 7 to 30,000 Daltons, more preferably 8,500 to 15,000 Daltons, especially about 10,000 Daltons. The method of goat immunisation can be intramuscular but other standard techniques such as subcutaneoue or intradermal adminstration can also be used. The purification process can also be completed by other commonly used fractionation action methods (caprylic acid for example) provided the total residue is used.

More particularly, the treatment typically employs a goat serum obtained in the following way.

### Example of Production of Goat Serum

A goat was inoculated by intramuscular injection with lysed HIV-3b virus suspended in a normal commercial supernate, using an intra-muscular injection of HIV-3b at a concentration of 10⁹ viral particles per ml. The virus was previously heat killed at 60°C for 30 minutes. Blood samples were drawn after an appropriate interval, such as two weeks, for initial assessment. In the optimised procedure, the goat is injected every week for four weeks, then at six weeks the animal is then bled to obtain the reagent.

Approximately 400 cc of blood is drawn from the goat under sterile technique. The area for needle extraction is shaved and prepared with betadine. An 18-gage needle is used to draw approximately 400 cc of blood from the animal. Of note is that the animal can tolerate approximately 400 cc of blood drawn without the animal suffering any untoward effects. The animal does not have to be sacrificed. The animal can then be re-bled in approximately 10 to 14 days after it replenishes its blood volume.

The presence of potentially useful antibodies was confirmed. Once the presence of such reagents was confirmed blood was then taken from the goat at between 4-6 weeks, and centrifuged to separate the serum. 300ml of serum was then filtered to remove large clots and particulate matter. The serum was then treated with supersaturated ammonium sulfate (45% solution at room temperature) to precipitate antibodies and other material. The resulting solution was centrifuged at 5000 rpm for five minutes, after which the supernatant fluid was removed. The precipitated immunoglobulin was resuspended in phosphate-buffered saline ('PBS buffer', see Sambrook et. al. 'Molecular cloning, A Laboratory Manual', 1989) sufficient to redissolve the precipitate.

The solution was then dialysed through a membrane with a molecular weight cut off of 10,000 Daltons. Dialysis was carried out in PBS buffer, changed every four hours over a period of 24 hours. Dialysis was carried out at 4°C.

After 24 hours of dialysis the contents of the dialysis bag were emptied into a sterile beaker. The solution was adjusted such that the mass per unit volume = 10 mg per ml. The dilution was carried out using PBS. The resulting solution was then filtered through a 0.2 micron filter into a sterile container. After filtration, the solution was aliquoted into single doses of 1ml and stored at -22°C prior to use.

The reagent is then ready for use.

Changes may be made in this procedure, such as for example by varying the concentration of the ammonium sulphate or switching to ther reagents. Similarly the dialysis cut-off need not be at 10,000 Daltons.

### THE INVENTION

As part of the treatments using the goat serum, it was noted that there were other beneficial results.

The findings of a significant clinical improvement in patients infected with HIV as well as those patients with diverse conditions such as multiple sclerosis and some cancers have the potential link that all these diseases are in chronic inflammatory states.

According to the present invention, there is provided the use of a polyclonal goat anti-HLA class II antibody as described in claim 1.

To our surprise we were able to demonstrate easily that the HIV immunised goats produced sera which is able to switch off the mixed lymphocyte response assay which is one of the classical activation assays used in vitro. Thinking that this might be some inherent property of goat serum, we were impressed when the non-injected goat sera failed to have any activity in these assays.

We also demonstrated that this activity is closely associated with activity against HLA class II antibodies. We do not know if this represents a molecular mimicry response or whether the goat sees HLA class II carried by the virus as it buds and is shed from infected cells, or that such HLA (MHC molecules) are shed seperately from the virus but co-purified so that antibodies are also made to such cell membrane components. Indeed, it has not escaped our attention that other cell membrane molecules such as chemokine receptors and related molecules may also be in the preparation and inducing antibody responses which are modulating the beneficial therapeutic effect. If it does however, it seem that anti-HLA II response are very dominant and we believe this may play a significant but not necessary a solitary role with regards to inducing the anti-inflammatory responses observed.

With the high titres of antibodies to HLA, both class II and class I in the sera, the ones with the highest levels of antibodies are the ones that switch off the mixed lymphocyte responses the best, and appear to have the best efficacy in the clinical situation.

To our surprise we found that the antibodies to FAS, a major apoptosis ligand, were extremely high. Indeed they correlated with the MHC Class II antibodies. Without being bound by theory; we hypothesise that high titre antibodies to FAS might lead to immediate apoptosis within a few minutes of the antibody attaching to the FAS antigen and the cells might be killed. This might lead to the inhibition of the toxic chemokines, which are thought to be involved in the disability of MS.

The main use of the present invention is for the use of a polyclonal goat anti-HLA class II antibody in the preparation, of a medicament for use in the treatment for diseases with inappropriately high levels of HLA. These are multiple sclerosis, rheumatoid arthritis, inflammatory diseases, autoimmune diseases and axonal or nerve damage.

The observed improvements in nervous function in those people with traumatic damaged nerves suggests a neuronal growth factor property and hence may be used for trauma, post infectious damage eg guillian-barre, malignancy damage etc, neuropathies associated with diabetes, alcoholism, poisoning with metals or other toxins etc.

Suitable antibodies can be raised by employing as immunogen a selection of antigens, preferably a cocktail of antigens. It is possible that the use of a range of different antigens give rise to antibody which recognises common structures of the antigens giving a stronger response in the patient. We hypothesise that a selection of HIV isolates will provide epitopes with minor variations in structure, and a pan-antibody will result.

Thus, to generate the serum, we prefer to employ a cocktail of different HIV viruses produced primarily in PBMCs, rather than use T-cells alone. The cocktail suitably contains 2, 3, 4, 5, 6 or more of such viruses. The viruses are preferably in the form of lysates. Examples of preferred lysates include the following HIV-1 isolates: 91US056, 92HT593, 92US723, 92US657, 92US660 and 92US714. Preferably the cocktail includes at least 1, 2, 3, 4, 5 or all 6 of these particular isolates.

Activation of cells, for example with Concanavalin A, can give advantages, and for example higher levels of anti-dopamine activity may be achieved using Con A. SHULA (non-activated) had no significant rise in the anti-dopamine R levels above baseline on O.D. Meanwhile, the HIV 3B (averages of 12 goats and rabbit) did. There was also a rise in Dopamine R levels between the Con-A activated PBMC cells in the cocktail.

Such antibodies can also be obtained using proteins containing the peptides isolated from HIV lysates, synthetic peptides. Antibodies to lysate can be obtained and tested.

Without being bound by our current theory, it seems that the antibody of this invention acts to suppress cell proliferation of the kind which is required by HIV or other conditions reliant on such an immune response. Thus, for example, the present invention finds application in the treatment of multiple sclerosis.

Being now aware of the significance of the anti-HLA II activity of the anti-body from the goat scrum, it now becomes possible to assess the probable utility of a range of such goat sera. A simple assay can assess the presence of anti-HLA II activity, and permit identification of candidate serum suited for administration to patients.

Based on the ability of the serum to dramatically inhibit the MLR, it is suggested that this serum may be acting as a strong anti-inflammatory agent. There are a number of mechanisms whereby this might be effected and inhibition of HLA recognition is one of the most likely.

Another mechanism of action may well be due to the fact that complement is involved in the activity of this goat plasma as it is the only activity known to be prevented by heat treatment. Complement may well be enabling patients' weakened defector mechanisms to be active. For example; both antibody targeted viral and tumour cell killing and cell mediated antibody directed killing both require complement and there is a total lack of effect or mechanism in the absence of complement.

In general, injection of antibodies into humans derived from a non-human host is counter-indicated. A strong immune response is normally mounted against the foreign antibodies themselves. However, surprisingly, it has been discovered that use of goat serum extract does not provoke the immune reactions which are anticipated with other foreign animal proteins. Injection of goat serum extract is tolerated both by immunosuppressed patients and normal individuals.

A killed virus is injected into a specifically identified goat, by intra-muscular injection, and allowed to incubate, thereafter a measured quantity of blood is drawn and modified accordingly.

The serum is optionally tested for the desired antibody activity, and optionally one or more of anti-fusion ability, neutralisation of the AIDS virus, its ability to enhance phagocytosis and its acceptability to the human body.

After inoculation of a selected goat with the HIV virus, an immune response after being exposed to a foreign protein antigen, was noted in accordance with earlier studies. The extracted serum was then further modified in order to prepare it for human use.

Approximately two hundred people aged 10 and above, of both sexes have been successfully treated with the medication, none of which have shown any relapses or suffered any side effects.

Treatment is given by means of a subcutaneous injection, in amounts varying between one/tenth and ten cc and is designed to deliver the medication as speedily as possible to the lymphatic system. The preferred dose for an HIV patient is usually 1 ml weekly or as required, given as a divided dose into both arms. Administration every 2 or 3 weeks becomes typical, then every 3 months. For cancer patients, 0.3 ml weekly seems best.

In most cases the treatment has been conducted once ever four weeks over a three month period. General observations of the same are as follows:
1. Moderate to severe depression was reversed in less than sixty minutes post injection.
2. Patients generally within two hours post injection regained their appetites and actively sought out food.
3. Within approximately two weeks of the first treatment the patients started to gain weight.
4. Independent laboratory reports confirmed that 4 to 6 weeks after the first treatment the viral loads and P24 values were dropping substantially and that CD4 and CD8 cells were increasing dramatically.
5. No side effects were observed.

It is important to note that the present medication, unlike current treatments, does not require the patient to maintain a strict hourly or daily regime and relies upon a simple injection being administered either weekly or monthly.

Preferably, the composition is purified and consists essentially only of a purified serum extract. In a further variation, antibodies may also be purified as a whole or selected and grouped in accordance with a disease-specific requirement from the complex serum or plasma mixture by conventional or any other suitable procedure, including, but not limited to, for example immunaffinity chromatography, salt precipitation, ion exchange chromatogrpahy, size chromatography, affinity chromatography, in combination as appropriate or desired.

A test dose is employed usually to see if the person develops an allergic reaction to the hyperimmune goat serum. An intradermal injection is followed by a wait of 30 minutes to see if there is an intermediate reaction which is manifested as oedema, erythemia, and itching. If this reaction is negative, then the assumption is that an immediate sensitivity reaction is most likely to occur. An allergic reaction does not preclude the person, however, from receiving a potential life-saving treatment because of a possible allergic reaction.

Administration of the composition of the invention may be by any suitable method such as by intravenous infusion, subcutaneously, intra-muscular injection, oral preparation, intraperitoneal and intravenous administration. The correct dosage will vary according to the particular formulation, the mode of application, and the particular situs, host and condition being treated. Other factors like age, body weight, sex, diet, time of administration, rate of excretion, condition of the host, drug combinations, reaction sensitivity and disease severity shall be taken into account. Administration can be carried out continuously or periodically within the maximum tolerated dose.

This product, unlike most other treatments, does not require the patient to maintain a strict hourly or daily pill-taking regime and relies upon the administration of a simple periodic injection. The immune systems of patients who were treated over two years ago and have remained with the project, seem to have stabilised and returned to normal operative levels.

Unlike existing drugs, which often need to be taken daily for the rest of the patients life, a typical treatment relies upon a simple injection being administered by a doctor either weekly or monthly. A normal treatment programme is of three months duration, with an anticipated follow up procedure at six months, twelve months and two years or as necessary should the virus reappear.

The composition of the present invention may be used with other drugs to provide a combination therapy. The other drugs may form part of the same composition, or be provided as a separate composition for administration at the same time.

Preferably, the composition of the present invention is treated by one or all of the following: precipitation with 45% ammonium sulfate, freezing at -70°C for 24 hours or microfiltration.

The recovery seen in many of the MS patients, alongside the elevated mood reported within the hour of receiving the treatment, has also prompted us to look for activity against receptors in the CNS which may be involved in nerve stimulation and possible regeneration. We have screened the various sera for activity against a number of antigens and have found activity against the dopamine receptor, serotonin receptor, Nerve growth factor receptor p75 and the chemokine CXCL10 (IP10).

Being now aware of the significance of the activity against dopamine receptor, serotonin receptor, Nerve growth factor receptor p75 or chemokine CXCL10 of the anti-body from the goat serum, it now becomes possible to assess the probable utility of a range of such goat sera. A simple assay can assess the presence of such activity, and permit identification of candidate serum suited for administration to patients. In particular, the combination of anti-FAS and/or anti-HLA antibodies may be important, along with antibody against one or more of dopamine receptor, serotonin receptor, Nerve growth factor receptor p75 or chemokine CXCL10 and thus assays might be directed at the various antibody activities to ensure their presence in the product.

### FIGURES

Figures 1 to 9 relate to determination of the presence of anti-HLA class II antibodies;
Figures 10 to 16 relate to MLR studies;

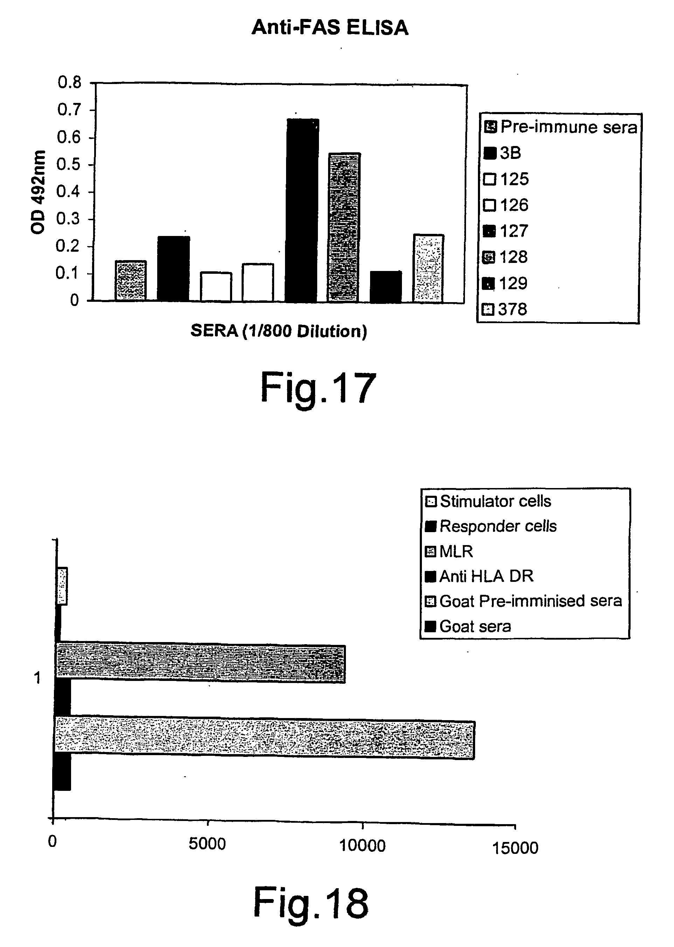

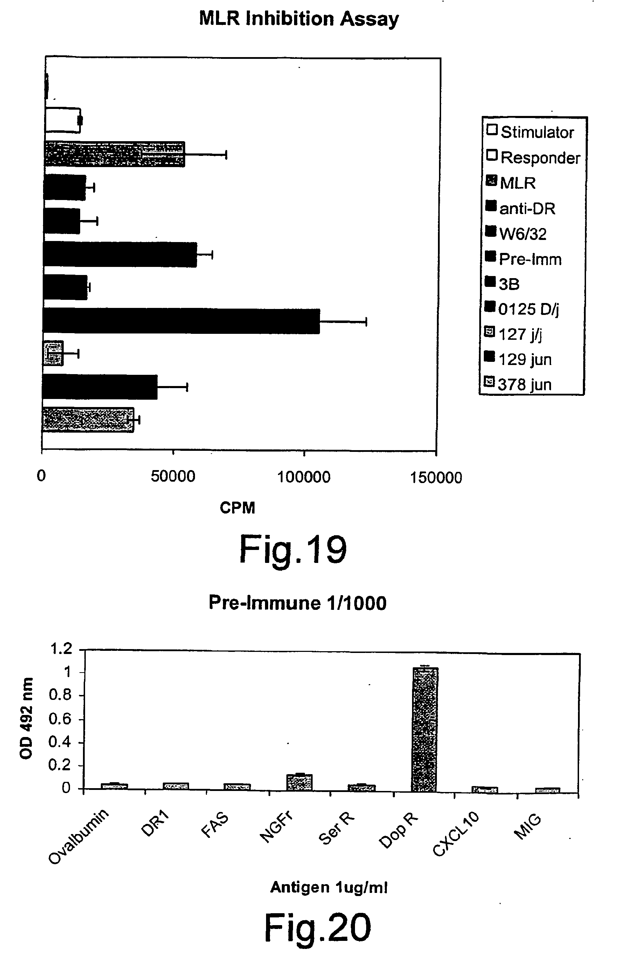

Figures 17 to 19 relate to determination of the presence of anti-FAS antibodies;

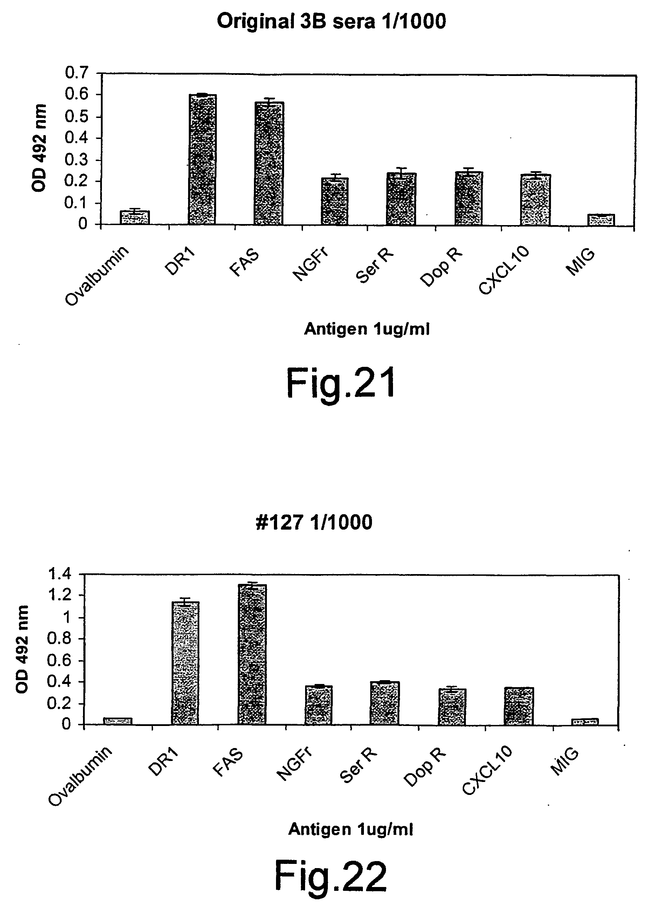

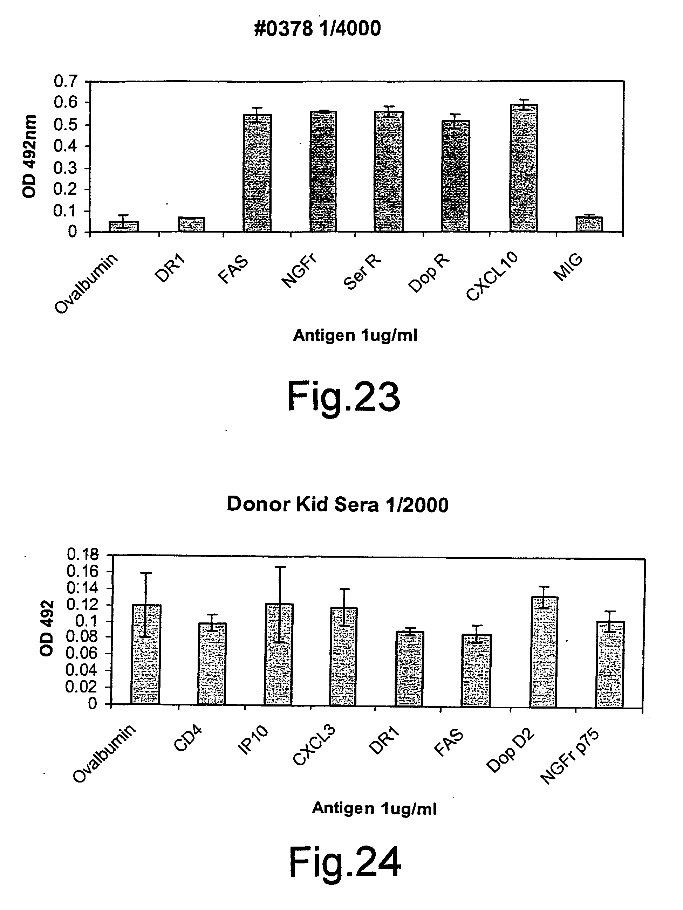

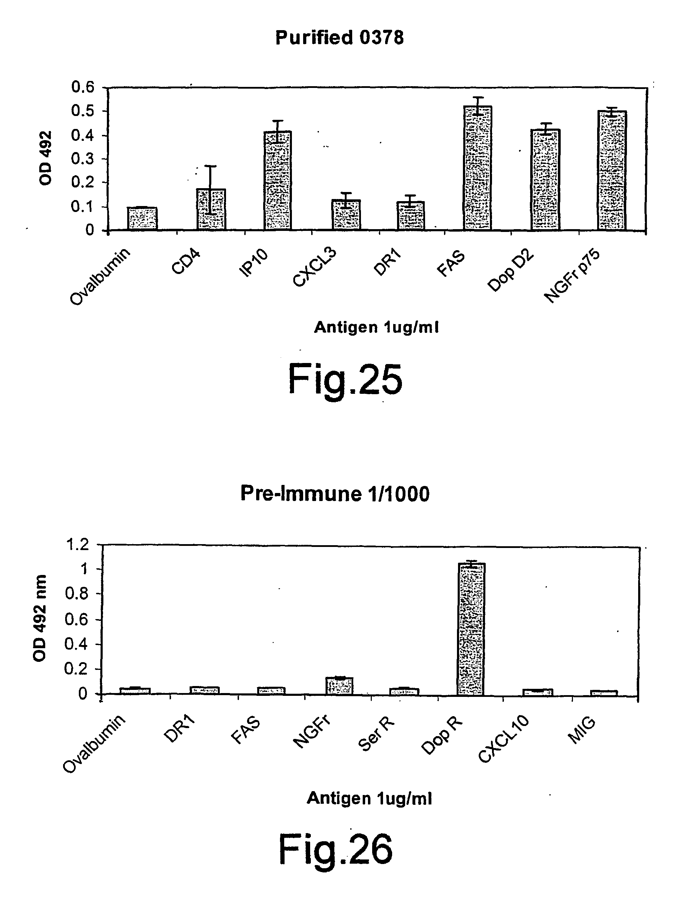

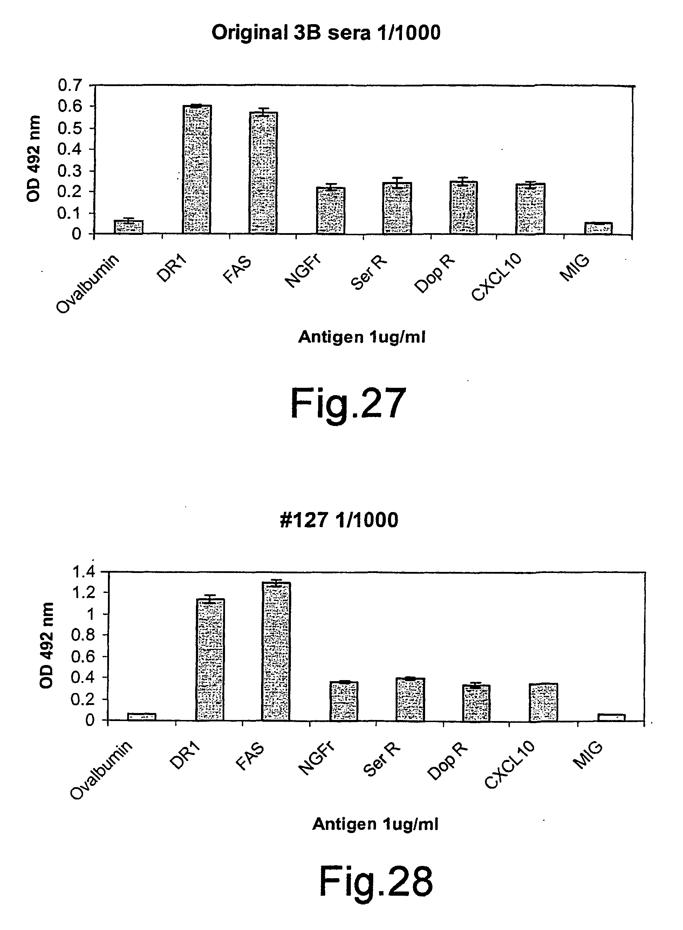

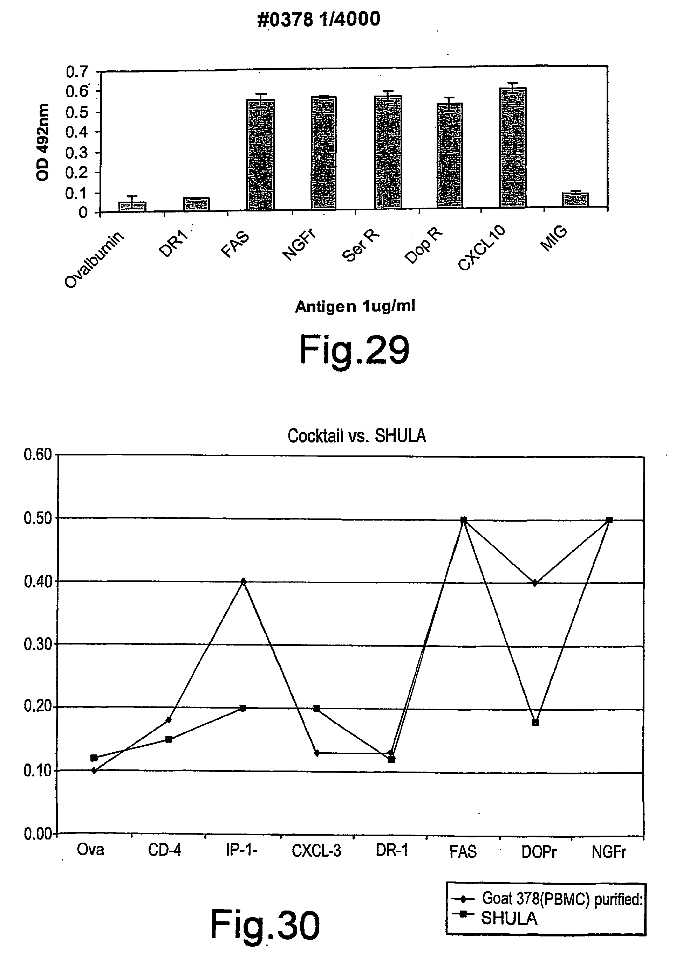

Figures 20 to 29 relate to determination of the presence of further antibodies; and
Figure 30 provides profiles for sera from goats immunised with different immunogens.

### EXAMPLES OF THE INVENTION

The following details represent our current procedure for preparing the antibody product.

### Example of Production of Goat Serum

A goat was inoculated by intra-muscular injection with lysed HIV viral cocktail and formulated with Freunds adjuvant. The virus was previously heat killed at 60°C for 30 minutes. Blood samples were drawn after an appropriate interval, such as two weeks, for initial assessment. In the optimised procedure, the goat is injected every week for four weeks, then at six weeks the animal is then bled to obtain the reagent.

Approximately 400 cc of blood is drawn from the goat under sterile technique. The area for needle extraction is shaved and prepared with betadine. An 18-gage needle is used to draw approximately 400 cc of blood from the animal. Of note is that the animal can tolerate approximately 400 cc of blood drawn without the animal suffering any untoward effects. The animal does not have to be sacrificed. The animal can then be re-bled in approximately 10 to 14 days after it replenishes its blood volume.

The presence of potentially useful antibodies was confirmed, having regard to the desired antibody activity. Once the presence of such reagents was confirmed, blood was then taken from the goat at between 4-6 weeks.

The base blood product in order to create the reagent is then centifruged to separate the serum. 300ml of serum was then filtered to remove large clots and particulate matter. The serum was then treated with supersaturated ammonium sulphate (45% solution at room temperature), to precipitate antibodies and other material. The resulting solution was centrifuged at 5000 rpm for five minutes, after which the supernatant fluid was removed. The precipitated immunoglobulin was re suspended in phosphate-buffered saline ('PBS buffer', see Sambrook et. al. 'Molecular cloning, A Laboratory Manual', 1989) sufficient to re- dissolve the precipitate.

The solution was then dialysed through a membrane with a molecular weight cut off of 10,000 Daltons. Dialysis was carried out in PBS buffer, changed every four hours over a period of 24 hours. Dialysis was carried out at 4°C.

After 24 hours of dialysis the contents of the dialysis bag were emptied into a sterile beaker. The solution was adjusted such that the mass per unit volume = 10 mg per ml. The dilution was carried out using PBS. The resulting solution was then filtered through a 0.2 micron filter into a sterile container. After filtration, the solution was aliquoted into single dosages of 1ml and stored at -22°C prior to use.

The reagent is then ready for use.

### Conversion of Plasma to Serum:

### Materials:

| Reagent | Formula | Grade | Quantity Required per Litre of Water |
|---|---|---|---|
| Sodium chloride | NaCl | USP/EP/BP | 8,76 g |
| Sodium dihydrogen orthophosphate | NaH₂PO₄ | USP/EP/BP | 0.2 g |
| Di-sodium hydrogen orthophosphate | Na₂HPO₄ | USP/EP/BP | 1.55 g |
| Dextran sulphate | | Sodium salt, molecular weight 500,000 | 100 g |
| Water-for-irrigation | N/A | Baxter | 1 litre |
| 1 x 150 mL sterilised Duran | N/A | N/A | N/A |
| 2 x 2 litre sterilised Duran | N/A | N/A | N/A |
| Magnetic stirrer plate & sterilised magnetic flea | N/A | N/A | N/A |
| 1 x Sartolab or Midisart filter | N/A | 0.2um | N/A |
| 6 x 1 L Stedim bags | | | |
| 1 x 100 mL sterile bag | N/A | N/A | N/A |

### Preparation of 100mg/mL dextran sulphate solution.

Dissolve the required quantity of sodium chloride, sodium dihydrogen orthophosphate and di-sodium hydrogen orthophosphate in 1 litre of water to make 1 litre of phosphate buffered saline (PBS) solution.

When completely dissolved slowly sprinkle 10 g of dextran sulphate into 100 mL of magnetically stirred PBS. Mix for a minimum of 10 minutes for complete solubilisation. Filter to 0.2um into a sterile container (e.g. 100 mL bag) and store at room temperature if not required for immediate use. This is the dextran sulphate stock solution.

### Conversion of plasma to serum.

Weigh out the required volume of plasma to be treated using a conversion factor of 1.0275, i.e. 1000 mL weighs 1027.5 g.

Add 10 mL of the dextran sulphate stock solution to each 1000 mL of the plasma (final concentration of 1mg/mL).

Magnetically stir at room temperature for 30 minutes. Transfer to 1 litre Stedim bags.

Centrifuge at 4000 - 4200 rpm (relative centrifugal force = 4910g) for a minimum of thirty minutes at 22° C.

Carefully aspirate the supernatant into a 1L Stedim bag and discard the precipitate.

Tare the required number of 1L Stedim bags.

Filter the supernatant to 0.2um into 1L Stedim bags. Each bag to contain approximately 500 ml of the filtered supernatant.

Record the volume of serum in each bag. Number and identify each bag.

If the serum is not to be processed immediately store refrigerated at 4 - 8°C for periods up to 7 days or at -20°C for longer periods.

### Preparation of 36% w/v Sodium Sulphate.

### Materials:

| Reagent | Formula | Grade | Quantity Required per Litre of Water |
|---|---|---|---|
| Anhydrous sodium sulphate | | USP/EP/BP | 360 g |
| Water-for-irrigation | N/A | Baxter | 1.5 litre |
| 1x2 litre sterilised Duran | N/A | N/A | N/A |
| Magnetic stirrer plate & sterilised magnetic flea | N/A | N/A | N/A |
| 1 x Sartolab or Midisart filter | N/A | 0.2um | N/A |
| 2 x 1 L Stedim bags | N/A | N/A | N/A |

Weigh out 1.5 litre of water (assume 1 mL = 1 gram) into the 2L sterilised Duran.

Warm water to 30-35 °C by placing the Duran into a pre-warmed incubator for at least 1 hour.

Introduce a sterile magnetic flea and place on the magnetic stirrer.

Slowly add the 360 g of sodium sulphate with stirring.

Keep stirring until the salt is completely dissolved and the solution is clear.

If the solution is not warm enough or allowed to cool, the salt will begin to crystallise out of solution. The salt may be resolubilised by heating the solution to 50°C.

Filter the 1 litre of the 36% w/v sodium sulphate solution to 0.2um into a 1-litre Stedim bag. Label the bag accordingly.

Filter the remaining 500 mL into a separate Stedim bag. Proceed to add a further 500 mL of water-for-injection by filtration and label this bag 18% w/v sodium sulphate.

Precipitation of Immunoglobulins from Serum Using Sodium Sulphate

Note. Plasma must be defibrinated by the dextran sulphate method prior to treatment with sodium sulphate.

### Materials:

| Reagent | Formula | Grade | Quantity |
|---|---|---|---|
| Filtered Serum | | | 1 litre |
| 36% sodium sulphate solution | N/A | | 1.5 litre |
| 18% sodium sulphate solution | N/A | | 1 litre |
| Phosphate buffered saline | N/A | | 2 litre |
| Water-for-irrigation | N/A | Baxter | N/A |
| Depth filter | N/A | N/A | N/A |
| 0.2um filter | N/A | 0.2um | N/A |
| Stedim bag for diafiltration | N/A | N/A | N/A |

Warm the filtered serum in the Stedim bags using the incubator set to 30-35°C. Take 2 x 5mL samples.

Add an equal volume of the warmed 36% sodium sulphate solution to the serum in the bags, e.g. 500 mL salt solution:500 mL serum.

Gently mix the salt: serum mixture (creamy white solution) by rocking the bags manually, or by placing on a rocker plate. Mix for 30 minutes.

Weigh and balance the bags and centrifuge at 4000 - 4200 rpm for a minimum of 30 minutes at 25-30°C.

Carefully aspirate the supernatant to waste. Try to avoid disturbing the precipitate. Take a 1 x 5mL sample of the supernatant.

To each bag of precipitate add a sufficient volume of the 18% w/v sodium sulphate to make-up the weight of each bag to around 1000 g.

Mix manually or by rocker for a minimum of 10 minutes to wash entrapped albumin out of the immunoglobulin precipitate.

Transfer the bags to the centrifuge and repeat centrifugation as above.

Carefully aspirate the supernatant to waste. Try to avoid disturbing the precipitate.

To each bag of precipitate add a sufficient volume of the phosphate buffered saline to make-up the weight of each bag to around 1000 g.

Mix manually or by rocker for a minimum of 10 minutes and transfer the bags to refrigeration for storage overnight.

Remove the bags from storage and ensure precipitate has resolubilised.

Filter to 0.2um into a suitable size diafiltration bag.

Take 2 x 5 mL samples.

### Bulk Formulation of Immunoglobulin Solution.

Set-up the ultrafiltration (UF) device with at least one 0.1m2 30,000 MWCO membrane.

Sanitise the system by recirculation of a 0.5 M - 1 M sodium hydroxide solution for a minimum of 30 minutes.

Drain the system of the sodium hydroxide solution and proceed to flush with water-for-irrigation until the pH of the water is neutral.

Connect the diafiltration bag containing the immunoglobulin solution to the UF.

Connect a bag containing PBS (10 x the volume of the immunoglobulin solution) via a peristaltic pump to the diafiltration bag.

Connect the retentate line to the diafiltration bag.

Connect the diafiltrate line to a waste bag.

Proceed to slowly process the product through the ultra filtration device, adjusting the inlet and outlet pressures to 2.0 bar inlet and 0.5 bar outlet, using the pump speed and valve adjustments.

Proceed to concentrate the immunoglobulin solution to around 50 g/L. This is calculated by taking into account the starting concentration and measuring the amount of liquid removed. For example:

Starting solution is 2 litres at 25 g/L = 50 g. Volume of solution removed in diafiltrate is 1 litre. Therefore concentration is 50/1 = 50 g/L.

Measure the diafiltrate flow rate and adjust the pump speed from the bag containing the PBS to equal this flow rate.

Proceed to continue diafiltration until 10 volumes of buffer have been exchanged, i.e. 1 litre of IgG solution requires 10 litres of PBS.

Drain the system of the concentrated diafiltered IgG solution into the diafiltration bag. Disconnect the bag and connect the PBS bag containing at least 1 litre of PBS directly to the system.

Recirculate the PBS rinse solution and recover this rinsate into the IgG solution-containing bag.

Proceed to filter to 0.2um the bulk formulated IgG solution into a pre-weighed Stedim bag.

When completed take a 1mL sample of the filtered solution and assess for protein concentration.

Weigh the bag and calculate the volume of IgG solution.

Using the protein concentration and volume of IgG calculate the volume of PBS required to dilute the solution to a final concentration of 10mg/mL, for example:
Volume of IgG = 1300 mL. Protein Concentration = 30 mg/mL
Total amount of IgG = 39000 mg
Requirement = 10 mg/mL final concentration.
Therefore final volume required = 39000/10 = 3900 mL. Current volume is 1300 mL. Therefore volume of PBS required to be added is 3900 - 1300 = 2600 mL.

Filter PBS into the bag containing the bulk formulated IgG solution to achieve the final required volume at 10mg/mL.

The reagent is ready for use and may be stored refrigerated for up to 1 week, or frozen to -20°C for longer storage requirements.

Changes may be made in these procedures, such as for example by varying the concentration of the sodium sulphate or switching to their reagents. Similarly the dialysis cut-off need not be at 10,000 Daltons.

### Mode of Activity

### Introduction

Private observations indicate that patients with AIDS given serum from HIV immunised goats appear to improve apparently dramatically in some cases, and we look for a potential mechanism. Although the goats are injected with HIV and the immune response is thought to be highly specific, this basis does not explain the observed benefit for patients with multiple sclerosis.

### Scientific Basis of the Study

Although Dalgleish and colleagues had discovered the CD4 receptor as the portal of entry for HIV several years ago, the classical interpretation that HIV kills CD4 cells which measurably decline as the infection progresses to AIDS was not a suitable explanation of the pathogenesis. Major observations that do not fit well with this interpretation include:
(1) The long time period from infection to the onset of AIDS (approximately a decade).
(2) The absence of disease in nearly all chimpanzees and in approximately 5% of HIV infected people. The chimpanzees have the same receptors and co-receptors as humans and the virus can be readily detected in infected chimps who do not drop their CD4 count.

Based on these observations, researchers have looked for the defining difference between those individuals who develop disease upon infection and those that do not. The major predictor of disease is the degree of immune activation following initial infection and the level at which it persists. In other words, the higher the immune activation, the shorter the time to the disease. A moderate degree of activation might lead to a longer time prior to the development of AIDS and an absence of immune activation even with virus replication is the role in chimpanzees and rare individuals.

It is important to point out that the term 'immune activation' in this context means pan immune activation i.e. all aspects of the immune system are activated, which includes all the subsets of T-cells as well as B-cells. There are only three recognised causes of pan-immune activation.

The first of these is hyper-variability in the antigen which HIV clearly exhibits, however the hyper-variability is detected in infected people and chimpanzees who do not progress.

The second possibility is a super antigen, which bypasses the antigen specific mechanism and causes activation of the entire immune system. In spite of an initial enthusiasm a decade ago, no convincing super antigen has been found to be associated with HIV that explains these findings. Westby and Dalgleish showed that the variability of the T-cell repertoire which had suggested the possibility of a super-antigen was entirely as a result of the decline in CD4 population and the differences might be explained by random CD4 destruction.

The only other explanation is that the body has seen a foreign cell or organ and mounted a chronic response to it. Clinically this often occurs after transplant and in particular bone marrow transplants and is known as chronic graft versus host (GVH) disease. In this scenario even the survival of a few foreign cells is enough to activate the entire immune system leading to aggressive auto-umnune responses similar to that seen in HIV infected patients. The clinical features of this disease are so remarkably similar to AIDS that prior to the discovery of the virus, a leading US Immunologist, Gene Shearer of the NIH (USA) suggested that the disease might be induced by foreign cells transmitted via blood transfusions or sexual intercourse. The discovery of the virus in the next few months and the transmission by Factor VIII (a cell free product) led to the significance of this observation being ignored.

The absence of another explanation for the pan activation led Dalgleish and Habeshaw to look for evidence that the virus might be able to mimic HLA Class I or Class II (which are the molecules that determine self and present antigens). Although some sequences of GP120 had already been published with minor homology to HLA, we were able to identify a major region of the HIV envelope (GP120) which had marked structural homology to both HLA Class I and HLA Class II. Elizabeth Hounsell (MRC) was able to model GP120 on the back of those HLA molecules whose structure was known in detail. Based on these molecules and sequences, Habeshaw and Hounsell predicted that the virus might be seen as "foreign" by people with HLA-B8 and also be seen as "self' by people with HLA-B27. It is now a matter of published fact that the only HLA differences of significance in a pan-HIV HLA marker study conducted in the UK by the MRC is that people with HLA-B8 developed disease at a far faster rate than the rest of the population and that the long term non-progressors or extremely slow progressors are HLA-B27.

Subsequent work has shown that:
(1) Killer T-cells mounted in response to a challenge with foreign cells (i.e. different HLA type) will also kill HIV infected self cells.
(2) That the envelope of the HIV virus known as GP120 (which we believe has structural homology to the HLA molecules that determine self), can bind peptides in a very similar manner to HLA. Moreover, T-cells raised to the peptides and the HLA which mimics GP120 will also respond to GP120 with the peptide but not to GP120 without the peptide suggesting that the ability to bind peptides may be very important in its confirmation and hence the ability to cause chronic immune activation in susceptible subjects.
(3) More recently we have shown that the region where peptides bind on HLA is present on GP120 and that if this part of the molecule is removed, as was kindly performed by Professor Sodroski at the Dana Faber in Boston, the peptides will no longer bind.

The impact of the above observations suggest that the immune activation of the system might in theory be switched off and that the disease therefore might not progress. There are two major observations in this regard, one of which was a clinical study as a result of this science in which patients who might no longer take AZT because of marrow failure were put on steroids, a classic heavy hitting anti-inflammatory agent which cannot be given for long periods without significant side effects. We were able to see marked improvement of very ill patients with classical features strongly resembling GVH disease with steroids and were able to show that the dose required to exert this effect was extremely high and not a practical proposition in the long term. More recently a group at Duke University (USA) have shown that the virus load falls in patients administered steroids for HIV which is now being done more and more to combat what is regarded as auto-immunity (a classical feature of chronic graft host disease). In short, there is a scientific basis that an anti-inflammatory agent that worked particularly on the HLA class I or class II induced pathways might be beneficial in HIV infection.

### Assays and Results

In order to see if the goat sera had any inflammatory properties, we added it to reactions formed by mixing the cells of two different individuals with different genetic backgrounds. To our surprise it was extremely effective at inhibiting this reaction. Using a large number of molecular antibodies, we were able to see that the only antibody that came close to the goat reactivity was an anti HLA class II. Anti-class I antibodies only partially reduced this reaction. Of great interest is that antibodies to the HIV V3 loop judged by most in the scientific community to be the main target for a vaccine, actually makes this pan activation worse. This finding might fit with the immune activation theory as the immune response to the virus actually gives growth and encouragement to the virus to "take off" and this might explain why vaccines aimed at this region are so ineffective.

Bearing in mind that magical properties have often been ascribed to goats and that sera from other animals can have unexpected activity in specific assays, we obtained non vaccinated goat sera which has no activity at all. We then had further sera from different goats with different vaccination schedules. To our surprise we can show that this sera only sees HIV envelope and HLA class II. Indeed, smaller portions of the virus which are known to have significant homology to HLA are not seen by the sera.

We were therefore extremely surprised to see a major difference between the cocktail injection and the one isolate injection, in that the repertoire of conserved HLA regions in the GP120 are actively recognised by sera of the cocktail injected goat.

The interpretation of this data is summarised by the fact that HIV injected goats produced a strong anti-MHC class II like response which can be broadened by using different isolates. The possible interpretation of how this comes about are as follows:
(1) The HIV envelope is so similar to HLA class II that it is recognised as such by the goats immune system which has a completely different HLA repertoire.
(2) The second possibility is that in the immunogen i.e. the virus preparation, the HLA which sits on the surface of the cells through which the virus buds, is being picked up and that the immune response is not to the virus but to the co-associated HLA.
(3) The third explanation involves a combination of the two explanations above. It is possible that the budding virus has fused with HLA as it prepared and that this combination is seen very strongly by the goats sera. A recent report shows that unless the virus bud through a cell with HLA and incorporates the HLA into the membrane then the virus remains uninfectious. This means it must have some HLA derive from the host cell to activate the cell entry mechanism.

Here we have shown that goats injected with HIV preparations make a strong antibody response to the HLA class II molecule. It is known that a number of diseases produce destructive inflammatory lesions purely because the cells involved over-express HLA class II. Although many types of autoimmune disease share this property, one of the best known example is that of multiple sclerosis. It may therefore be the case that the immune response of the goat breaks tolerance to these molecules and that this process might allow a strong anti-inflammatory process to occur in vivo which might be associate with clinical benefit. There will be obvious advantages if this was the case over long term high dose steroid usage if chronic administration of the goat serum if free of significant side-effects.

The anti-HIV response which is strongly anti-HLA class II may well provide a vital link as to the true requirement of an anti-AIDS vaccine and it is our intention to dissect this response to produce a candidate HIV vaccine.

HIV-1 Immunised Goat Sera and anti-inflammatory properties.

### Introduction

Goat sera from animals immunised with HIV-3B viral lysate or an NIH viral cocktail of 6 different HIV-1 isolates were provided. Some of the sera has previously been used as part of experimental treatment regimens to good effect, however the mechanism by which it acts is unknown. To determine a mechanism of action we screened the sera on ELISA plates against HIV-1 gp120, HLA-DR1 and chosen peptides from different regions of the virus surface glycoprotein, some bearing sequence homology with HLA.

### Materials and Methods

### Sera and antibodies

Samples of the original HIV-3B immunised goat sera that had been used for patient treatment along with samples from a number of HIV-3B viral lysate immunised goats (animals #0125, #0126, #0127, #0128, #0129 immunised on 14 September 1999, 21 September 1999 and again on the 29 September and 07 November 2000) were provided as well as information concerning bleed and immunisation dates. For the ELISA , we simply chose to screen sera samples taken from production bleeds at the later stages (30th Nov 2000 and 01 Dec 2000). Animal #378 was immunised with an NIH Viral cocktail consisting of the following HIV isolates ( 92HT593, 92US657, 92US660, 92US714, 92US723, 91US056 ). Immunisations were performed on the 7th and 28tb November. Sera taken from a production bleed on January 25th 2001 was used for ELISA screening. Control goat sera was also provided. Alongside the sera we included anti-human HLA-DR and anti-human HLA-DR, DP and DQ (Pharmingen) in both ELISA and Mixed Lymphocyte reaction studies and mouse anti-gp 120 IgG (EVA3047) specific for the V3 domain of HIV-i IIJb (IRIQRGPGR) obtained from Dr J Laman through Dr Harvey Holmes (MRC AIDS reagents programme, National Institute for Biological Standards and Control, Potter's Bar, UK) during the MLR study.

### Peptides and Proteins

Recombinant HIV-i IIIB gp12O was produced in Chinese hamster ovary cells and provided by Dr J Raina via Dr Harvey Holmes at the MRC AIDS reagents program. Recombinant HLA-DR 1 was the same protein provided by Prof. Don C. Wiley used during peptide binding experiments. HIV-1 peptides used in the study are listed in Table 1. Peptides ARP7022, ARP7 10, ARP740-23, -28, -42, -44, -45, -46 and - 47 were obtained from Dr Harvey Holmes at the MRC AIDS reagents programme. Control peptide P12 represents a scrambled sequence of the CS region of HIV-1 gyp12O and was provided by Prof. E. F. Hounsell, School of Biological and Chemical Sciences, Birkbeck College London, UK. Peptides were stored in aliquots at -20°C until use.

### ELISAs

ELISAs were performed following a protocol similar to that described by Brown et al. (J. Immunological methods 1997, 200:79-88). Peptides and proteins were mixed with 0.05M pH 9.6 Carbonate/Bicarbonate binding buffer at 16µg/ml and 1µg/ml respectively and coated in duplicate onto Immulon 4 LIBX high binding Microtiter plates ( Dynex Technologies, INC. 14340 Sullyfield Circle, Chantilly, VA 20151-1683, USA) overnight at 40°C. Wells were blocked using 5 mg/ml casein in PBS and left overnight at 40°C. Goat sera were diluted 1/500 and 1/1000 times in PBS/0.25% Casein whilst purified antibodies were diluted 1/1000 and 1/3000 times respectively and incubated for 1h at 37°C. Wells were washed 3 times with PBS/0.05% Tween 20 using a Wellwash 4 machine (Denley).

Immobilised goat antibodies were detected using peroxidase conjugated Mouse Monoclonal Anti-Goat/Sheep IgG clone GT-34 (SIGMA) diluted 1/1000 in PBS/0.25% Casein/0.01% Tween 20 and anti-HLA antibodies detected using a Goat anti-mouse IgG Peroxidase conjugate (SIGMA) incubated for 1h at 37°C. Following another round of washes, freshly prepared O-phenylenediamine dihydrocbloride substrate (OPD; SIGMA) was incubated in the wells at room temperature in the dark and reactions stopped after 15 minutes by addition of 50 µl/well 2.5M H₂SO₄. The OD values were measured at 492nm using a Microplate Reader.

### Table 1. Peptides Used during assays

ARP7022 : (DQQLLGIWGCSGKLICTTAVPWNC)
24 residue peptide from a conserved region of HIV gp41 (593-616) recognised by most European and African HIV positive sera. Control peptide.
ARP710: (VKIEPLGVAPTKAKRRVVQREKR)
23 residue peptide derived from the conserved C-terminal (CS) domain of HIV gp120 (486-508) leading to the gp120/41 cleavage site. Contains structural homology with HLA
ARP740/23: (RPWSTQLLLNGSLAEEEVV)
20 residue peptide derived from the C2 region of gp12O (252-271). Contains sequence homology with the HLA DR4 β-chain.
ARP740/28: (NTRKRIRIQRGPGRAFVTIG) (302-321) 20 residue peptide derived from the V3 loopHlV-1 gp120
ARP740/42: (GQIRCSSNITGLLLTRDGGNS) (438-458) Contains homology with DR-β1 chain.
ARP740/44: (NNESEIFRLGGGDMRDNWRS) (459-478) Contains sequence homology with HLA-A2
ARP740/45: (GQDMRDMWRSELYKYKWKI) (469-488)
   Sequence recognised by M38, an antibody which cross-reacts with HLA-C and the C5 region.
ARP740/46: (ELYKYKWKIEPLGVAPTKA) (469-478)
   20 residue peptide derived from the C5 terminus of gp12O. Contains the first 3 residues of homology at the C-terminus.
ARP740/47: (EPLGVAPTKAKRRVVQREKR) (479-498)
   20 residue peptide derived from the C5 region of gp12O. Contains structural homology with HLA.
P12. (RAKTVERKVERRK)
   Scrambled sequence of the CS domain of HIV gp12O supplied by E. Hounsell. Not recognised by WV positive sera. Control sequence.

### Mixed Lymphocyte Reaction Inhibition assays

### Blood Donors

HLA Class II mismatched blood samples were provided with consent from donors through Liz Buckland of the South Thames Blood transfusion service, Tooting, London.

### PBMC Preparation

Freshly drawn venous blood was diluted in Hanks' Balanced Salt Solution (HBSS; SIGMA), carefully overlaid on Histopaque (SIGMA) and centrifuged at 800g for 25 minutes at 20°C. PBMCs were harvested from the density interface with a Pasteur pipette, washed 3 times in HBSS and counted using a Beckman Coulter counter.

### Mixed Lymphocyte inhibition assays

For a given MLR, PBMCs from two HLA class-II mismatched individuals were resuspended in RPMI 1640 medium containing 10% heat inactivated human AB sera (SIGMA), 4 nM L-glutamine, Penicillin (100U/ml) and Streptomycin (100µg/ml) (SIGMA) and designated either stimulator or responder cells. Cells were plated in triplicates with siimulators at 1 x cells/well and responders at 1 x 10∼ cells/well to give a 10:1 Responder - Stimulator ratio. To determine any inhibitory effects on cell proliferation exacted by goat sera and various antibodies, 3µl of undiluted goat sera or 1µl of purified antibody were added to the wells containing the mixed cell cultures and incubated at 37°C in 5% CO2 for 6 days. Cultures were pulsed with 1µCi of tritiated methyl-thymidine (³H-Thd; Amersham) on day 5, 18h before cell harvest. Cells were harvested using a Tomtec Harvester 96 Mach III cell harvester onto glass fibre filter mats and ³H-Thd incorporation measured using a Wallac 1450 microbeta liquid scintillation counter. Results are shown as mean counts per minute (cpm).

### Results

### Anti-HLA class II antibodies present in goat sera

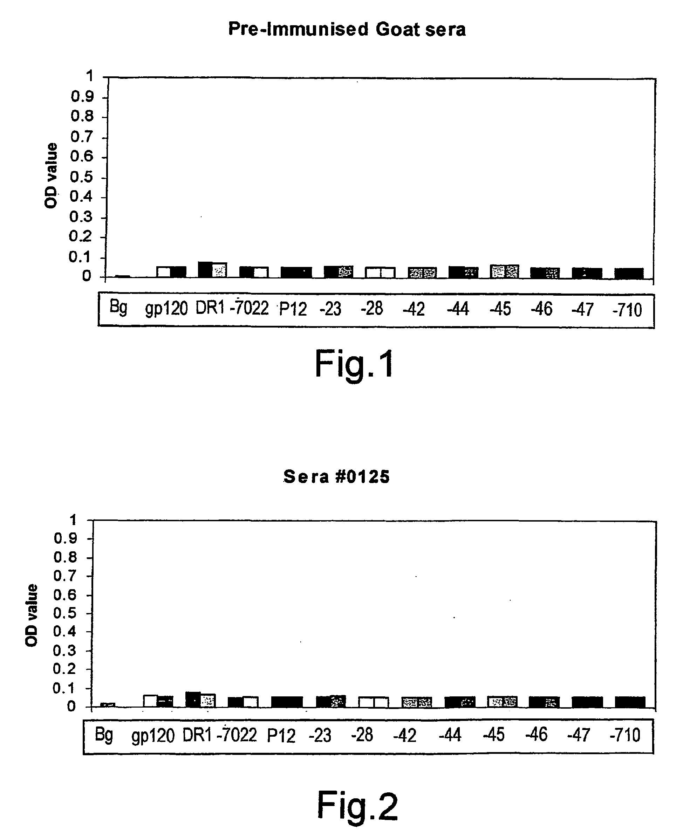

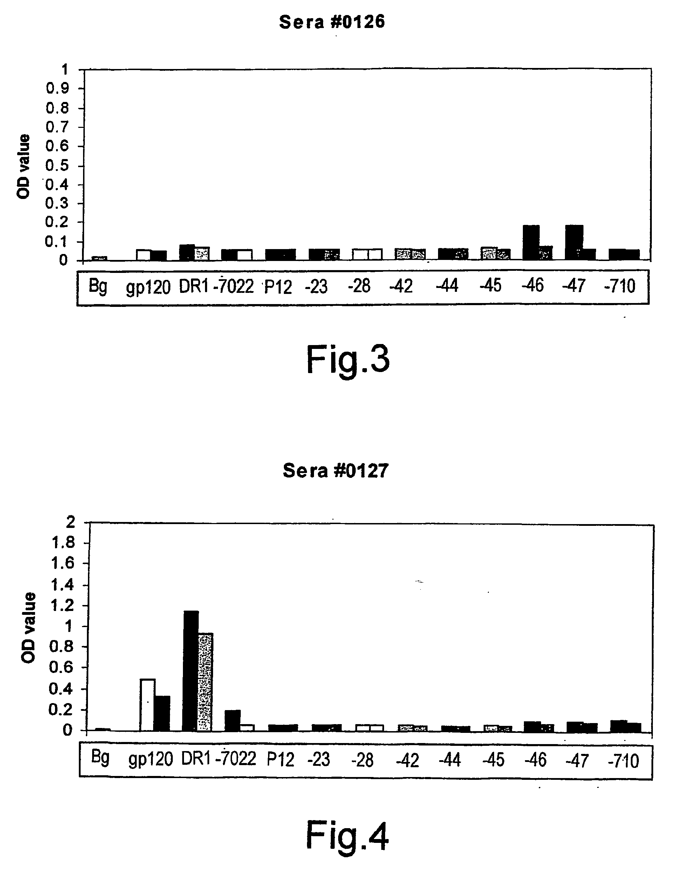

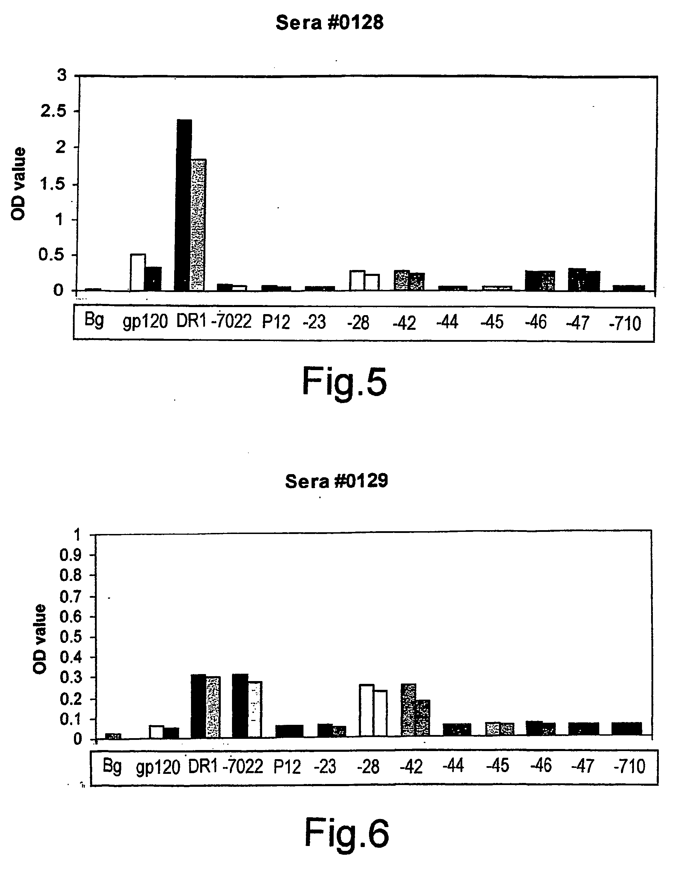

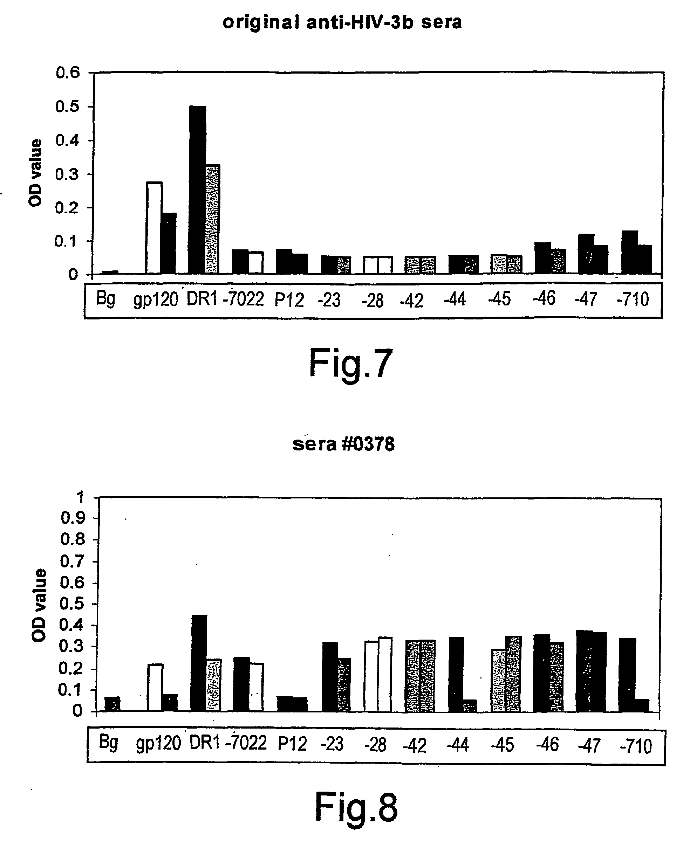

We investigated the extent to which the differing goat sera might recognise HIV-1 gp12O peptides taken from different regions across HIV-1 gp12O, many bearing sequence and structural homology to HLA (Table 1). We also included soluble HIV-1 gp12O and HLA-DR1 in these assays. The results are shown in figures 1 to 9. Bg represents background levels. An OD level above 0.1 was taken as a positive result given the lack of reactivity to any of the antigens seen with the pre-immunised sera.

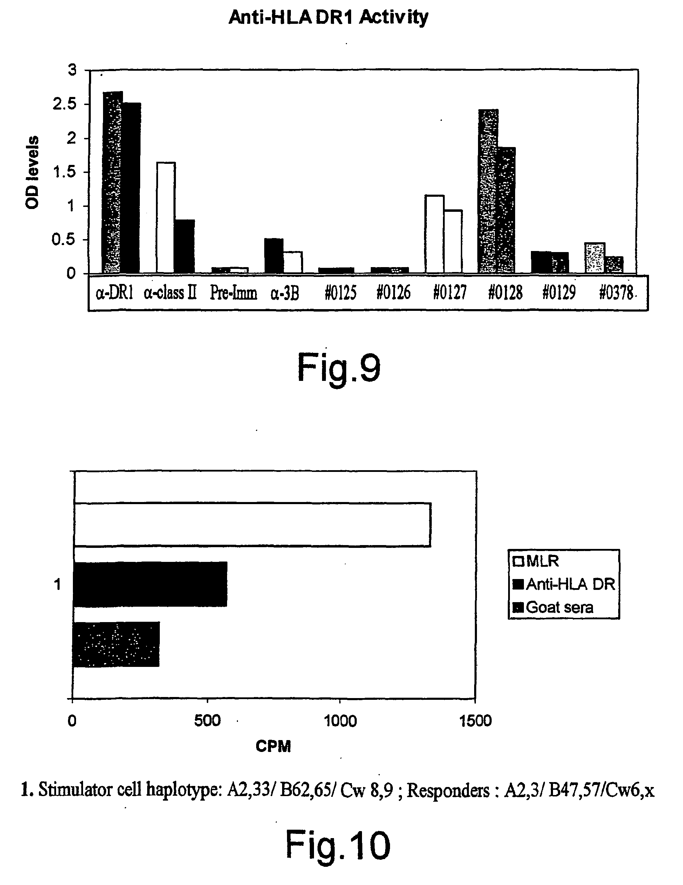

The different sera gave varying results but generally pointed towards a trend to react with HLA-DR1. The pre-immunised sera was not reactive with any of the screened peptides or proteins, and sera #0125 and #0126 did not show any great reactivity. However all the other sera demonstrated high levels of reactivity to HLA-DR1, in particular #0127 and #0128 which had very high reactivity. The reactivity to HLA-DR 1 is compared in figure 9 alongside anti-human HLA-DR and anti-human HLA DR, DQ and DP for clarity.

The anti-HLA activity is interesting for the reason that it most likely represents activity against the HLA molecules from the very cells the virus was cultured in prior to immunisation. As HIV tends to drag along more HLA molecules in its envelope than it expresses gp12O molecules, particularly HLA class II molecules which it upregulates in cells, the combined anti-gp 120 and anti-HLA class II activity may be what represents a good protective response to HIV. It may be the case of certain sera, particularly #0127 and #0128, that a strong "allo"-response was induced in these goats through immunisation with virus which carried high-levels of Class II molecules on its surface.

Sera #0378 from the goat immunised with the NIH Viral cocktail demonstrated the best all round cross-reactivity to multiple HIV-i peptides with anti-HLA DR1 levels approximately the same as that of the original anti-LILV-3B goat sera suggesting this sera might perform better overall against HIV. This sera reacts very well with a range of HIV-1 peptides bearing homology with HLA. None of the sera reacted with the scrambled control sequence P12 demonstrating that the reactivity was specific for the LIIV peptides. Sera #0127 and #0128 represent the best sera in terms of binding to HIV-i gp12O whilst containing very high amounts of anti-HLA DR activity.

### MLR Inhibition by Goat Sera

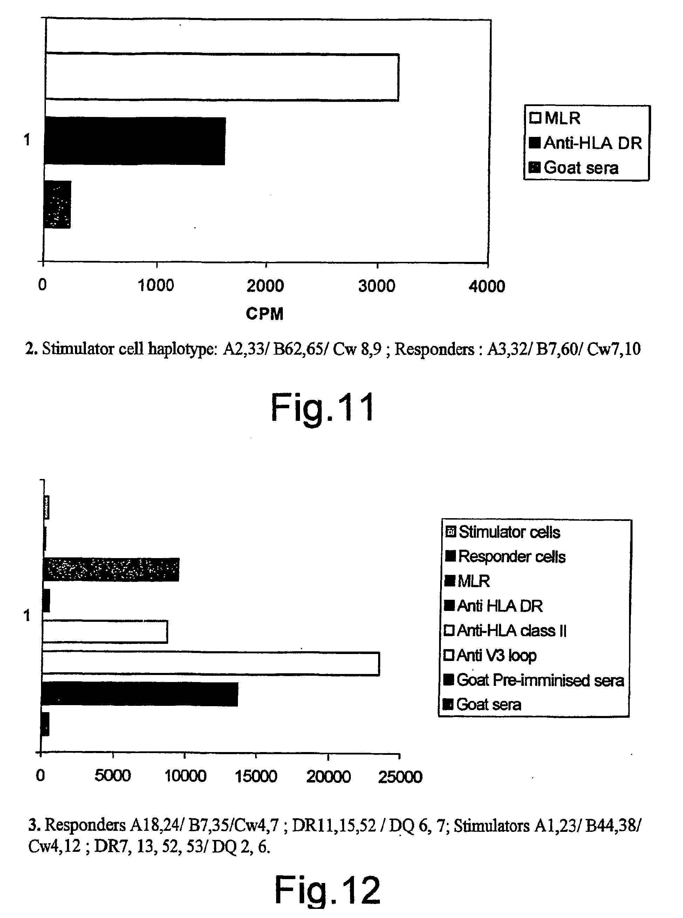

With the MLRs of figures 10 and 11 we used cells from random mismatched individuals. Although these did not necessarily produce the greatest proliferation (at the time there were problems with the media), the trend appeared demonstrating inhibition of proliferation during MLRs by goat sera (Original HIV-3B immunised sera used in each case). Subsequently we began to use HLA-class II mismatched cells (fgiure 12) which produced better proliferation. As with the anti-HLA DR antibody, goat sera succeeds in completely inhibiting cell proliferation and might appear to have anti-inflammatory activity.

Judging by what we have seen from the ELISA results, it is highly likely the anti-HLA DR antibodies are responsible for the inhibitory effects. We stress the role of anti-HLA DR rather that anti-class II as a whole as it appears that the broad range antibody does not inhibit proliferation anywhere near as effectively as anti-HLA DR alone. Why this might happen is mysterious, however it might be that HLA-DR represents the chief class II antigen involved in inflammatory activity and antibodies against this may result in signalling within cells to inhibit replication. The goat sera might therefore appear to act as an anti-inflammatory agent, suppressing cell proliferation. This might be predictive of a positive outcome in the case of HIV-1 infection where virus-induced cellular activation is essential for virus replication. Although at the surface it may appear counter-productive, the introduction of a mild immunosuppressive agent might act to inhibit virus replication and control disease as HIV, unlike related retroviruses HTLV- 1 or HTLV-2, does not immortalise cells and cause cancer so is entirely dependent on cell activation for proliferation.

To date no known T-cell superantigen has been identified for HIV which leads to the point of how a virus which appears relatively ill-equipped to activate the cells it infects can manage to cause disease. We are investigating the theory that virus induced alloactivation is at the root, be it associated with the viral associated HLA molecules or the molecular mimicry seen between HIV and HLA. The goat sera possibly contributes to patient wellbeing simply by limiting this pan-activation. Observations along similar lines have been made in a paper by Saifuddin M et al. (Clin. Exp. Immunology 2000, 221: 324-331) who demonstrated the importance of HLA class II molecules, in particular HLA-DR, in viral replication. They noted that antibodies to HLA class II molecules inhibited virus expression in class II expressing cells and suggested the involvement of the inducible MHC class II transactivator (CIITA) in enhancement of HIV-1 transcription. We might effectively be seeing these effects in the case of the goat sera. Take this in comparison to the anti-V3 loop antibody, which causes increased proliferation and might therefore be counterproductive in the overall scheme of things. The highly variable V3 loop region of gp12O not only permits viral escape mutants but also acts as a decoy for the immune system for fuither rounds of activation. Unfortunately preoccupation with this region is ultimately beneficial for the virus and keeps the immune response away from more conserved regions, against which activity might be more useful.

If the anti-inflammatory effects are indeed the main mode of activity of the sera, than it might come in useful to a variety of other conditions where hyper-activityand cell proliferation are at the root of disease symptoms, for example multiple sclerosis. For future experiments it is important to repeat the ELISAs for all the sera to confirm the extent of the anti-HLA and gp12O activity. In this case we are very interested in sera #0378 from the animal immunised with the NIH viral cocktail as it reacts effectively with many of the HIV peptides bearing homology to HLA antigens as well as HLA-DR1.

### Further Experiments not part of the invention

The following data comes from similar experiments to those previously undertaken, where we identified anti-HLA antibodies in the goat sera which has been used as a form of treatment for volunteers suffering from multiple sclerosis and AIDS when conventional therapy was no longer suitable. Our work involves investigating a proposed anti-inflammatory mechanism of action for the goat sera in the recovery of these patients following treatment. This was previously proposed when the anti-HLA antibodies were noticed. However we now add a role for anti-FAS antibodies present in the goat sera, given their ability to induce apoptosis within hours to cells expressing the FAS receptor.

FAS/APO-1 (CD95) is noticeably present at high quantity on active lymphocytes, signalling through which can induce that cell to commit suicide following crosslinking with either FAS- Ligand or an anti-FAS antibody. The role of these molecules is extremely important not only in the cytotoxic immune response for killing infected cells but also in downregulation of the immune response after the antigen has disappeared to prevent constant proliferation of cells which might be detrimental to the host. Therefore this pathway is deemed vital for the maintaining a balanced immune response.

In the case of HIV infection, the immune system is seen to be abnormally active giving plenty of space for viral replication as it requires activated cells for this process. In multiple sclerosis patients, highly activated cells cross the blood brain barrier and are responsible for damage to the myelin sheath, resulting in plaque formation and loss of neural function.

The anti-inflammatory mechanism mediated through the activity of anti-HLA antibodies (anti-HLA DR at least) present in the goat sera might be the mechanism behind patient recovery. We have now found anti-FAS antibodies present in the goat sera to varying extents and propose to combine the roles of anti-HLA and anti-FAS antibodies in this proposed anti-inflammatory mechanism for the mode of activity in both multiple sclerosis and AIDS patients. We propose that these antibodies are targeting the most highly active cells resulting in their death whilst anti-HLA antibodies dampen any immune activity. There might then follow dramatically reduced cytokine and chemokine production resulting in the termination of immune attack on the myelin in the case of multiple sclerosis or reduced viral load in the case of HIV infected patients given the lack of activated cells and the death of cells secreting or about to secrete virus.

### Experimental Work

We began to look for anti-FAS antibodies given observations that the goat sera from the responders was damaging the cells when added into Mixed lymphocyte reactions (MLRs). MLRs are the result of lymphocytes from 2 different people with different HLA types reacting in a non-specific manner resulting in lymphocyte proliferation and increased turnover. We feel this is a good system to test anti-inflammatory antibodies as autoimmune reactions are all aimed at inappropriate expressing of self-peptides on HLA class 2 molecules.

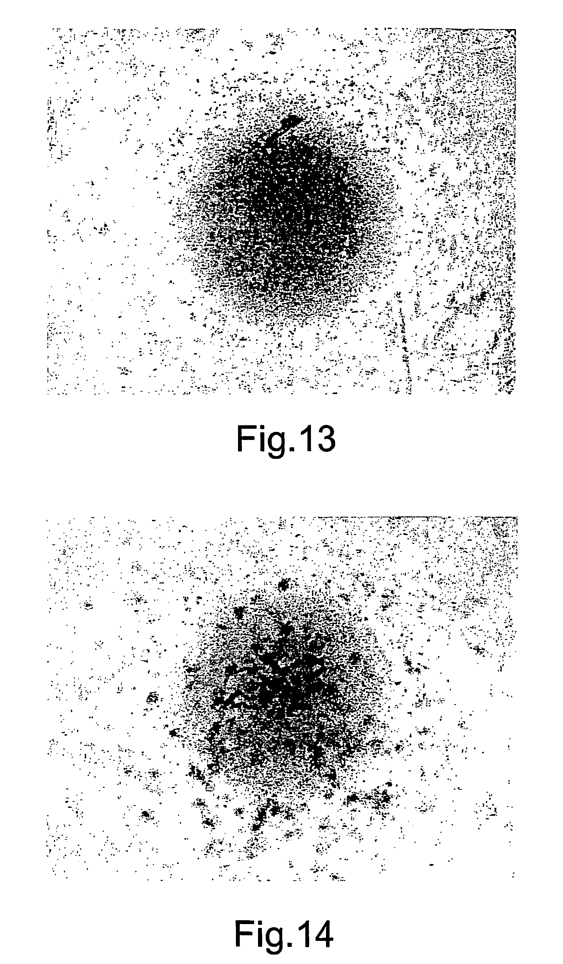

Figures 13 to 16 are photos taken from some recent MLRs, with and without sera.
Figure 13 is a photo of a mixed lymphocyte reaction after 72 hours.
Figure 14 is a MLR mixed with Pre-Immunised sera followed for 72 hours.

In all cases there are no significant changes from that with the MLR itself. In fact, the pre-immune sera promotes the MLR.

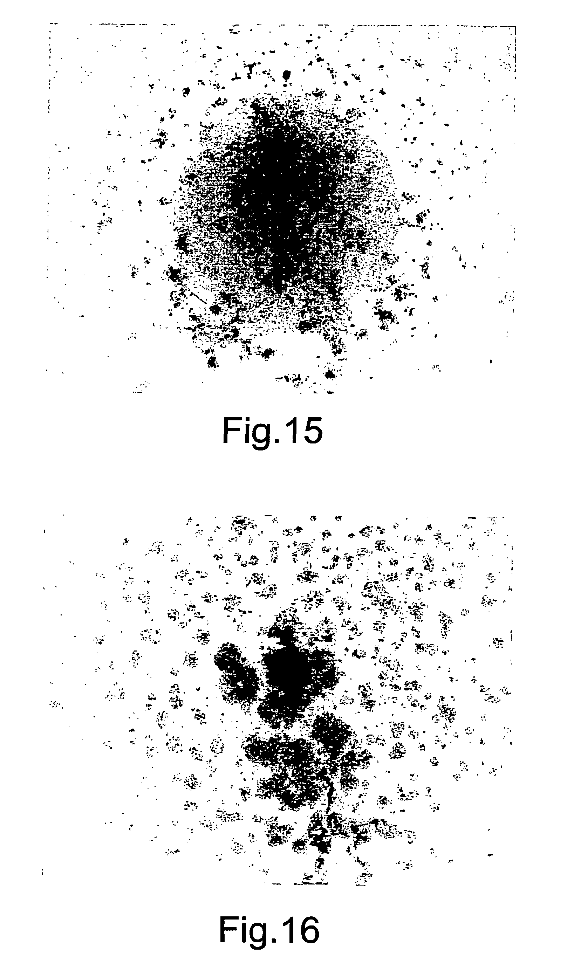

Figure 15 is an MLR with sera #0125 added. This sera came from a goat which was deemed unresponsive to immunisation in that low or negligible HLA and/or FAS antibodies were present, something which correlated with our previous ELISA results when we identified anti-HLA DR in the responders.

Figure 16 is an MLR with sera #0127 added, which has been shown to be a very good responder. There is significant cell destruction of many of the PBMCs in the MLR. This result is similar for all the strong responders along with the sera preparation currently being used for treatment.

We decided to look for an antibody capable of inducing destruction of highly active cells, as might be the case in an MLR. We therefore looked for the presence of an antibody against FAS which might be capable of crosslinking the FAS receptor and inducing the signals necessary for apoptosis to occur. Consequently we performed an ELISA against FAS which demonstrated the presence of such antibodies in the sera of the goats which were deemed the best responders and which complemented the previous results with the anti-HLA antibodies.

These results, along with our previous data, correlate well with what we have seen in our MLR results with reduced cell proliferation being due to cellular destruction.

From recent ELISA results we undertook to identify further possible active components in the goat sera which has been used as a form of treatment for volunteers suffering from multiple sclerosis (MS) and AIDS where conventional therapy was no longer suitable.

Below the results from an ELISA screen at a 1 in 1000 dilution of the pre-immunised sera. The antigens screened are Ovalbumin, HLA-DR1 (DR1), Fas, Nerve growth factor receptor p75 (NGFr), Serotonin receptor (Ser R), Dopamine receptor (Dop R), CXCL 10 and Monokine induced by interferon-gamma (MIG).

It is already apparent that the antibody to the dopamine receptor is present at high concentrations in the sera whereas there is no activity to any of the other antigens. However there is activity against many of these antigens in the original 3B sera, which has previously been used for treatment, and sera 0127 produced from a goat immunised with HIV IIIb produced in a T-cell line, including against activity against NGFr, the dopamine receptor and CXCL10.

The activity against HLA and FAS is again apparent and in keeping with the anti-inflammatory mechanism we have already proposed. In keeping with this theme we might now add activity against the CXCL 10 and the nerve growth factor receptor p75 (NGF R p75). Anti-sera against CXCL 10 has recently been shown to be beneficial by greatly reducing disease severity in the murine model of multiple sclerosis through reducing CD4+ T-cell and macrophage invasion of the CNS, diminishing expression of the TH1 cytokine IFN-γ and increasing remyelination. This is interesting given that local cells in inflammatory lesions commonly produce CXCL 10 which binds wand attracts inflammatory TH1 cells via the receptor CXCR3. Therefore neutralisation of this chemokine or blocking of its receptor by antibodies in the goat serum might well be part of the anti-inflammatory mechanism of action we have proposed for the goat serum.

Meanwhile antibodies to the low affinity Nerve growth factor Receptor NGF R p75 are associated with cell death in HIV infected monocytes and macrophages through blocking NGF from reaching the receptor, which again represents another anti-inflammatory mechanism. Of interest we have found the strongest levels of antibodies against these receptors to be present in sera 0378 produced through immunising goats with a cocktail of 6 different HIV viruses produced primarily in PBMCs rather than T-cells alone. The activity remains very high even with a 1 in 4000 dilution.

We therefore add the activity against these antigens, in keeping with the anti-inflammatory mechanism of action proposed for the sera in the case of both diseases. We also add activity to the dopamine and serotonin receptors as being responsible for the sense of well being associated with the treatment.

We screened many new goats (707-718) which have recently been vaccinated with the HIV along with the pre-immune sera from these goats. Although none of them show antibody against the dopamine receptor like the original pre-immune sera shown above, some of the goats have on average a higher level of such antibody by comparison with the goats from Wales which never had Rabies vaccines. The higher levels seen in the original pre-immune sample could be due to exposure to some environmental factor at some time in the past followed by a strong response.

## Claims

1. The use of a polyclonal goat anti-HLA class II antibody in the preparation of a medicament for use in the treatment of a disease in a human involving a proliferative immune response, wherein the disease is an inflammatory disease, an autoimmune disease, axonal or nerve damage, and
wherein the polyclonal goat anti-HLA class II antibody is raised upon challenge by HIV.

2. The use of claim 1, wherein the antibody has anti-HLA class II and anti-HIV activity.

3. The use of claim 1 wherein the disease is multiple sclerosis.

4. The use of claim 1 wherein the disease is rheumatoid arthritis.

5. The use of claim 1 wherein the disease is neuritis.

## Patentansprüche

1. Verwendung eines polyklonalen Anti-HLA-Ziegen-Antikörpers der Klasse II bei der Herstellung eines Medikaments zur Verwendung bei der Behandlung einer Krankheit bei einem Menschen, die eine proliferative Immunantwort mit sich bringt, wobei die Krankheit eine Entzündungskrankheit, eine Autoimmunkrankheit, eine axonale oder Nervenschädigung ist und
wobei der polyklonale Anti-HLA-Ziegen-Antikörper der Klasse II auf die Provokation mit HIV hin gezüchtet wird.

2. Verwendung nach Anspruch 1, wobei der Antikörper eine Anti-HLA-Aktivität der Klasse II und eine HIV-Aktivität aufweist.

3. Verwendung nach Anspruch 1, wobei die Krankheit die Multiple Sklerose ist.

4. Verwendung nach Anspruch 1, wobei die Krankheit die rheumatoide Arthritis ist.

5. Verwendung nach Anspruch 1 wobei die Krankheit die Neuritis ist.

## Revendications

1. Utilisation d'un anticorps polyclonal de chèvre anti-HLA de classe II dans la préparation d'un médicament pour l'utilisation dans le traitement d'une maladie chez un être humain faisant intervenir une réponse immunitaire proliferative, dans laquelle la maladie est une maladie inflammatoire, une maladie auto-immune, une lésion axonale ou nerveuse, et
dans laquelle l'anticorps polyclonal de chèvre anti-HLA de classe II est suscité par provocation par le VIH.

2. Utilisation selon la revendication 1, dans laquelle l'anticorps a une activité anti-HLA de classe II et anti-VIH.

3. Utilisation selon la revendication 1, dans laquelle la maladie est une sclérose en plaques.

4. Utilisation selon la revendication 1, dans laquelle la maladie est une polyarthrite rhumatoïde.

5. Utilisation selon la revendication 1, dans laquelle la maladie est une névrite.
